Europäisches Patentamt

(19) European Patent Office

Office européen des brevets

(11) Publication number: **0 108 069**
**B1**

(12) **EUROPEAN PATENT SPECIFICATION**

(45) Date of publication of patent specification: **11.03.87**

(51) Int. Cl.⁴: **A 61 M 5/20**

(21) Application number: **83900076.7**

(22) Date of filing: **13.12.82**

(86) International application number:
**PCT/SE82/00423**

(87) International publication number:
**WO 83/02062 23.06.83 Gazette 83/15**

(54) **INJECTION DEVICE.**

(30) Priority: **14.12.81 SE 8107458**

(43) Date of publication of application:
**16.05.84 Bulletin 84/20**

(45) Publication of the grant of the patent:
**11.03.87 Bulletin 87/11**

(84) Designated Contracting States:
**AT BE CH DE FR GB LI NL SE**

(56) References cited:
**EP-A-0 004 365**
**US-A-3 720 211**
**US-A-4 108 177**

(73) Proprietor: **BLOMBERG, Anders**
**PL. 6238 Höglanda**
**S-434 00 Kungsbacka (SE)**

(72) Inventor: **BLOMBERG, Anders**
**PL. 6238 Höglanda**
**S-434 00 Kungsbacka (SE)**

(74) Representative: **Mossmark, Anders et al**
**Patentbyran WEST-PATENT AB Stora Nygatan 15**
**S-411 08 Göteborg (SE)**

Courier Press, Leamington Spa, England.

## Description

### Technical Field

The present invention refers to an injection device for medical purposes, for injection by using a manual hypodermic syringe, the syringe including a barrel portion which at one end has a syringe needle and at the other end a flange, and a plunger portion having a plunger displaceable in the barrel portion, said plunger performing movements, enabling medicine to be drawn into or injected from the barrel portion, and having a gripping means connected with the plunger by means of a plunger rod, said injection device comprising an opening, through which the injection syringe can at least partly be introduced into a cavity, and an inner part which is provided with a holding device for the barrel portion, the holding device being capable of holding the barrel portion against movements in its longitudinal direction relative to said inner part; a displacement device for displacing the plunger portion relative to the barrel portion the inner part being moveable between a rear position with the syringe needle positioned inside an opening and an advanced position with the syringe needle pushed outside said opening in order to insert the needle into body tissue; and an outer part, in which an electric motor is positioned, said electric motor being connected to said displacement device by means of a transmission mechanism.

When treating certain diseases it is required that the patient regularly, often daily, is supplied with medical material by means of injections. One such disease is diabetes, affecting many men where it is important that the patient to a high degree can attend to the injection of insulin into the body by themselves. However, complications are connected with this disease and blindness is one of them. Many of the insulin dependent diabetics also have other additional diseases, which complicate the insulin injections, for instance a slight hemiplegia, rheumatism or Parkinson's disease. The fact that the frequency of diabetes also increases with age makes loading and injection of insulin with a conventional hypodermic syringe to a great problem.

### Technical Background

The majority of the diabetics use a completely manual hypodermic syringe, the so called disposable hypodermic syringe, which a blind or handicapped person has considerable difficulties to load with the prescribed amount of insulin and then insert the needle into the skin followed by injection. From US—A—4 104 177 an automatic injector device is prior known. This device enables automatized injection, but this device will not be a satisfactory aid for a handicapped person. This known device has no means for automatic loading the hypodermic syringe with medicine and therefore the loading must take place in a conventional manual manner before insertion of the syringe into the injector device.

### Technical Problem

The object of the present invention is to provide an injection device with which loading as well as inserting and injecting is accomplished without any demands on the users sight or manipulation ability.

### The Solution

The object is achieved by means of an injection device according to the present invention, which is characterized in that said opening through which the syringe can be introduced is said opening through which the syringe needle is advanced in use, the holding device is moveable into a releasing position, in which the barrel portion is released, the displacement device is capable of displacing the plunger portion in both directions relative to the barrel portion between a fore and a rear displacement position and in the fore displacement position is in a releasing position for the plunger portion and in the remaining displacement positions is capable of moving the plunger portion in either direction, and that the electric motor is controlled by means of an electronic control device, for moving the displacement device in a sequence determined by means of the control device for loading the injection syringe as well as insertion and injection.

### Brief Description of Drawings

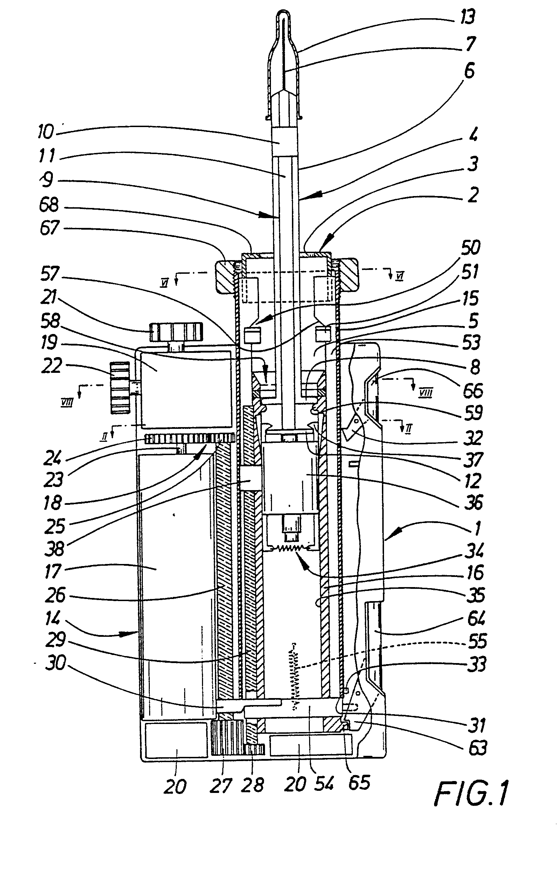

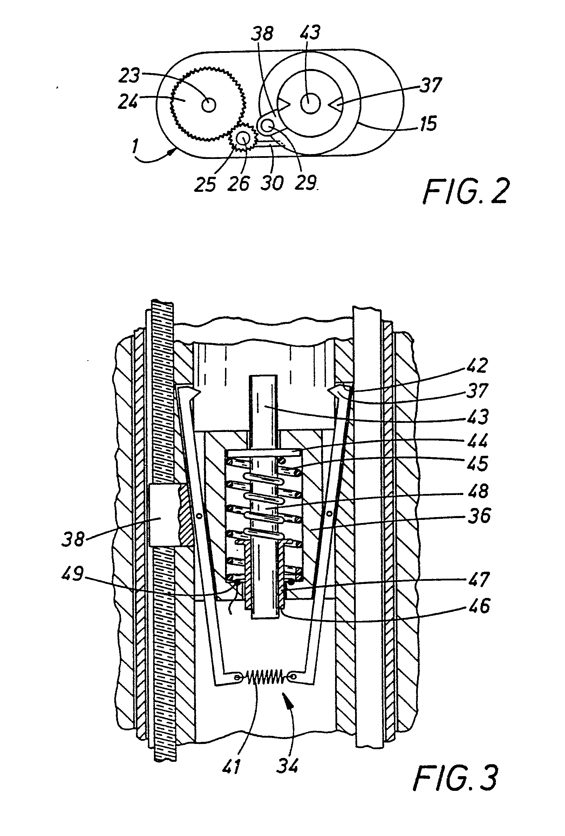

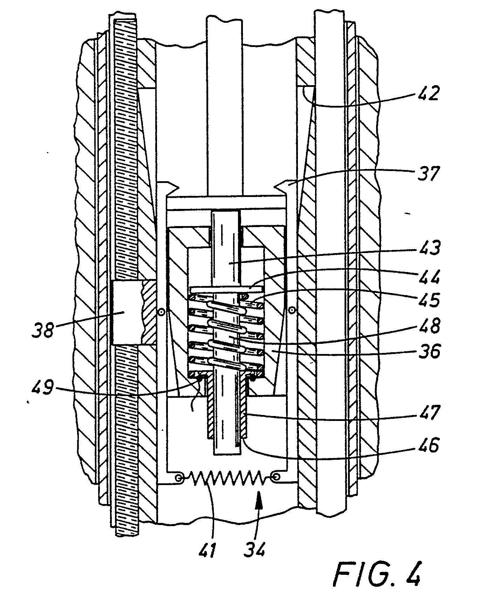

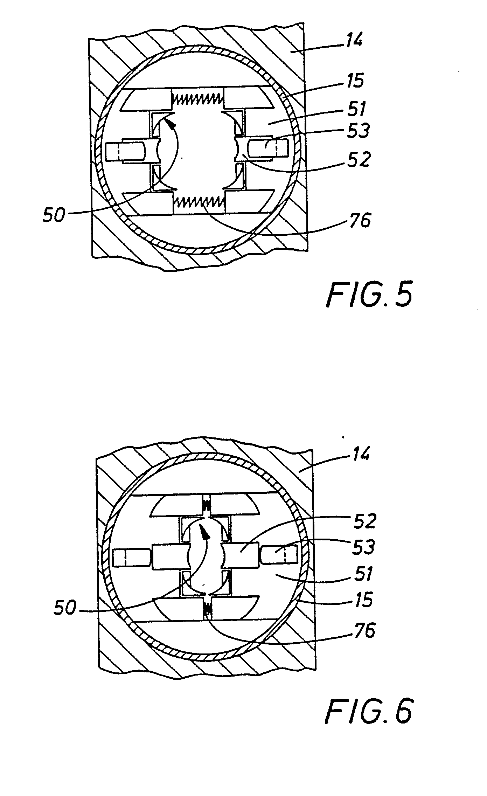

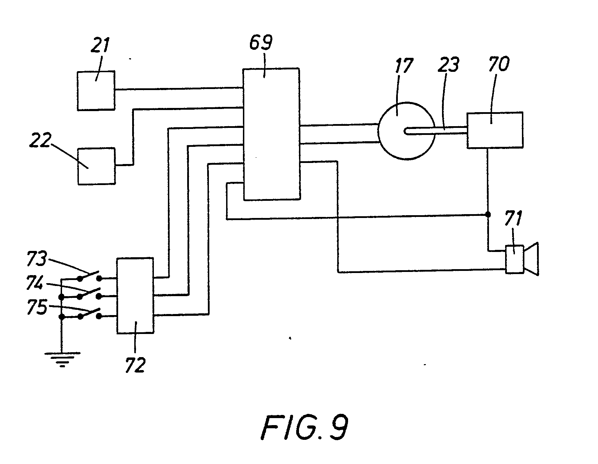

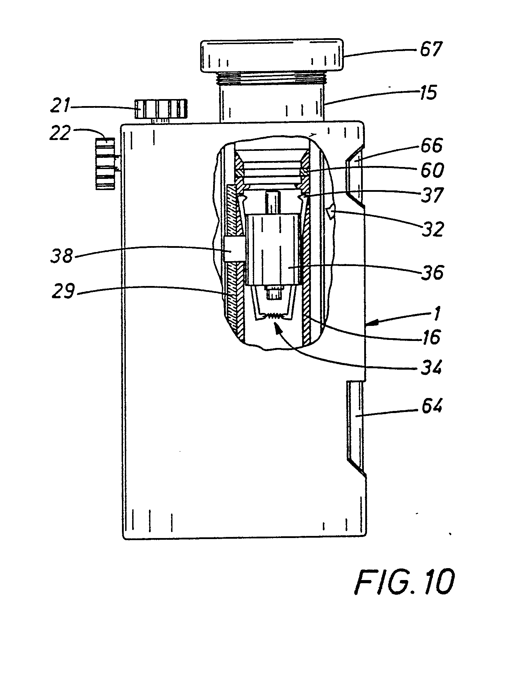

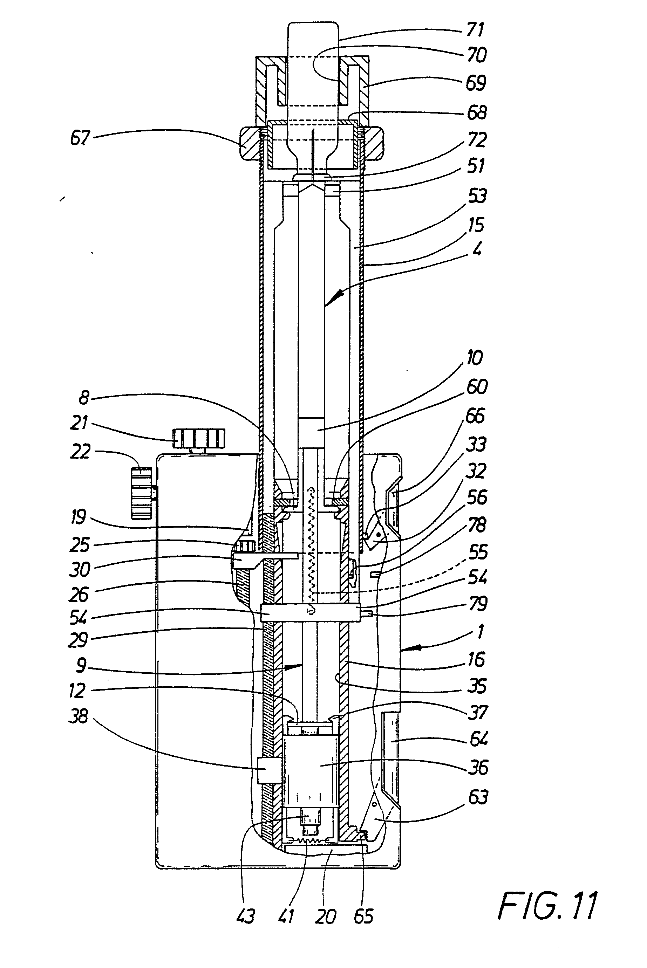

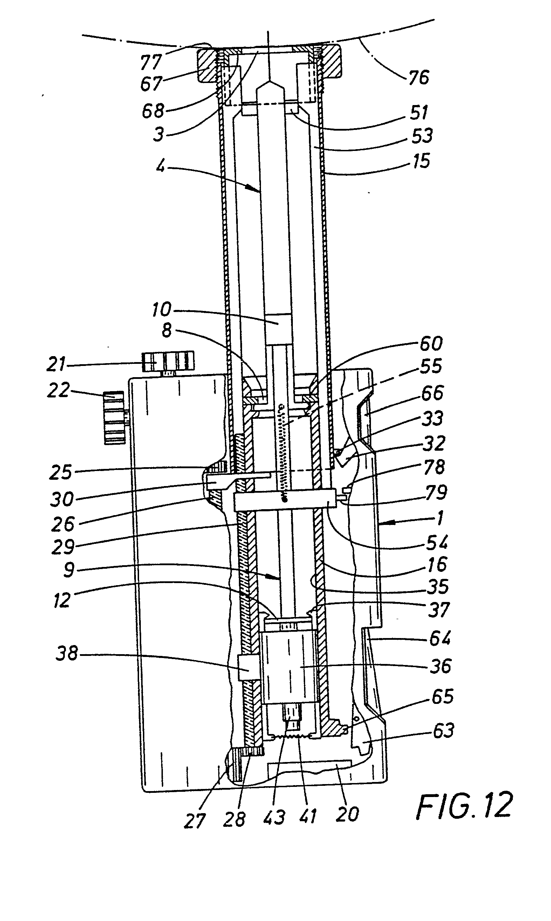

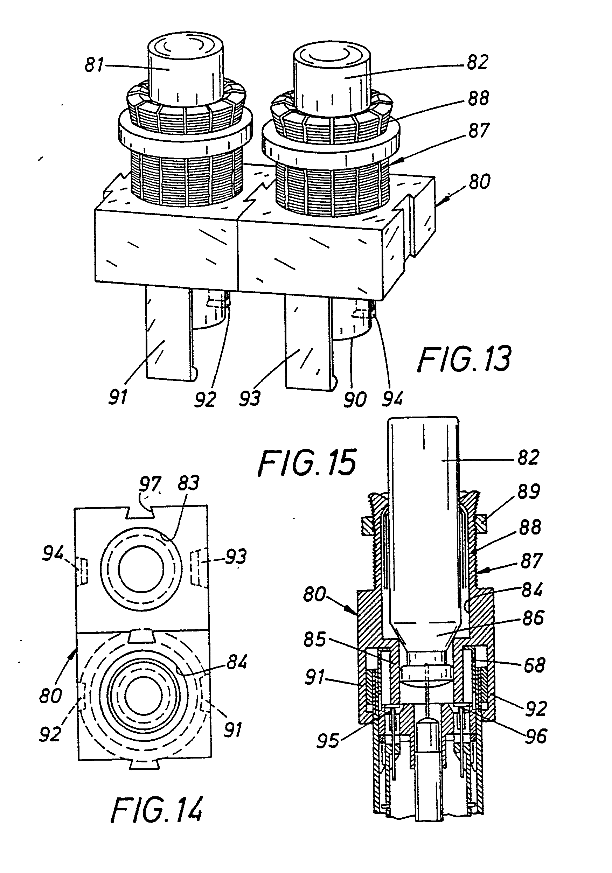

The invention will be more closely described in the following with an embodiment, reference being made to the enclosed drawings, in which Fig. 1 shows a partly broken view of the injection device according to the invention with an attached disposable hypodermic syringe; Fig. 2 is a schematic section along the line II—II in Fig. 1 of the injection device; Figs. 3 and 4 show in a larger scale a part view of a mechanism in the injection device for displacing the plunger part of the disposable hypodermic syringe in two different positions; Figs. 5 and 6 show a supporting mechanism for the disposable hypodermic syringe located in the upper part in the injection device in two different positions in a view along the line VI—VI in Fig. 1; Figs. 7 and 8 show a section through the injection device along the line VIII—VIII in Fig. 1, showing a holding means for the body of the disposable syringe. Fig. 9 shows schematically a block schedule over an electronic controlling device forming a part of the injection device. Fig. 10 shows in a partly broken view the injection device in an initial position before the disposable hypodermic syringe has been inserted into the injection device. Fig. 11 shows the hypodermic syringe injecting into a body tissue after being inserted into the tissue. Fig. 12 shows the hypodermic syringe injecting after insertion in a body tissue; Fig. 13 shows a perspective view of a bottle holder for two injection bottles; Fig. 14 is a horizontal view through the bottle holder while Fig. 15 is a partly broken view showing the bottle holder adapted to the upper end of the injection device.

Best Mode of Carrying Out the Invention

In Figs. 1 and 2 the principal construction of the injection device 1 is evident. In its upper part 2 it comprises an opening 3, through which a hypodermic syringe 4 of a disposable type can be introduced into a cavity 5 in the injection device. The disposable syringe is of a standard type and mainly consisting of a barrel portion 6 which forms a cylindrical part and in its upper part has a syringe needle 7 and in its rear part is provided with a flange part 8 in the form of two laterally directed flanges. The disposable syringe 4 furthermore has a plunger part 9 consisting of a plunger 10, a plunger rod 11 and a gripping plate 12, situated at the outer end of the plunger parts. The gripping plate 12 is connected to the plunger 10 which is movable inside the syringe body 4 in order to aspirate respectively push medical material through the syringe needle 7. The syringe needle is protected by a detachable top-cover attached to the barrel portion. The injection device 1 is built up by three relative each other movable main parts, viz. an outer part 14, which forms a house for the injection device, a middle part moveable relative the house and a movable inner part 16, movable relative the middle part, e.g. ½—1 cm. In the inner part 16 the disposable syringe is held in a manner which will be further described in the following. The house 14 has a cylindrically made bore like a guide for the middle portion, out of which the middle portion can be pulled out to an extended position, which will also be described more closely in the following. In the house 14 there are besides the middle portion 15 and the inner portion 16 with attached inner parts also situated an electrical motor 17 and the main parts of a transmission mechanism 18 for achieving at least some of the working movements of the injection device.

Furthermore the house encloses an electronic controlling device 19, made to control the motor 17 according to certain predetermined sequences in a manner which will be more closely described in the following. In the bottom of the house 14 there are accumulators 20 supplying the motor 17 and the controlling device with electric current. On the house there are also two adjustable wheels 21, 22 for adjusting the two different amounts of fluent medicine to be injected. For the sake of simplicity it is assumed in the present embodiment that the medicine is insulin, of which certain patients require two different kinds to be loaded in the syringe.

The amount of insulin of one kind is set by the wheel 21 and the amount of insulin of the other kind is adjusted by the other wheel 22. For the sake of simplicity, in the description of handling with reference to Figs. 1, 10—12, one case is indicated when there is only one sort, while Figs. 13, 14 and 15 show the case with two sorts of insulin. The wheel 22 that is intended for the other sort can for example be set in a zero position. The electrical motor is in the shown example a D.C. motor with a rotating terminal shaft 23, which extends parallel with the moving directions of the middle portion 15 and the inner portion 16. The D.C. motor 17 can be provided with a capsulated gear train (not shown) for gearing down of the rotations of the motor. The transmission mechanism 18 is constituted of a pinion 24 on the outgoing shaft 23 and a pinion 25 standing in a gear connection with the pinion 24 and is fixed on the other end of a threaded driving shaft 26, which has a rotating bearing in the house 14 and exhibits in its other end a pinion 27 of a relatively large width. This pinion 27 stands in gear connection with another pinion 28 fixed on the end of a screw shaft 29 which is threaded in the same direction as the driving shaft 26. The screw shaft 29 has a rotating bearing in the wall to the inner portion 16. The driving shaft 26 is connected to a driving joggle 30 which has a bore with internal threads and is guided in a not shown slot in the inner portion 16 through which the driving joggle 30 is made to move along the driving shaft 26 when the driving shaft is rotating. The driving joggle 30 acts on an edge, e.g. the lower edge 31 of the middle tube in order to lift it while moving upwards and to displace the middle tube in the direction out of the house 14 to an outer position shown in Figs. 11 and 12. In the outer position it is hooked with a springloaded pawl having a rotatable bearing in the house 14. The pawl is brought to act on a projecting portion 33 on the middle tube. When the driving shaft 26 is brought to rotate in the opposite direction, which makes the driving joggle to move downwards, the middle tube 15 thus remains in its outer position.

The inner part 16 has a displacement device 34 for the gripping plate 12 of the plunger portion 9. The displacement device 34 is displaceable in a bore 35 of the inner part 16 between a fore position shown in Fig. 10 and a back position shown in Fig. 11. The displacement device 34 is consists of a mainly cylindrical body 36 in which two gripping means are rotatably attached. They are made to hold the gripping plate 12 and bring it within the displacement movements of the displacement device.

The construction of the displacement device 34 is best shown in Fig. 4, showing a broken partial view of the injection device in an enlarged scale. In Fig. 3 the displacement device 34 is shown in its fore end position, in which the gripping means 37 is positioned in a releasing position and in Fig. 4 in a somewhat displaced position in which the gripping means 37 is in a holding position for the gripping plate 12, and in this way the gripping means are positioned during the displacement movements of the displacement device except the position shown in Fig. 3. The displacement movements of the displacement device are achieved with rotation of the screw shaft 29 acting on a nut 38 or more precisely a bore internally threaded, projecting as a part from the cylindrical body 36. The gripping means 37 are each positioned in a groove 39 in the cylindrical body and each one has bearings with its own pivot 40 in the cylindrical body. The two grooves 39 are successively enlarged over the pivot 40 in order to let the gripping means 37 perform a pendling movement through action by a tensioning spring 41 connecting two gripping means with each other below the cylindrical body. As the inner portion 16 in its wall has two widenings 42 positioned opposite to each other intended to take up the gripping means 37 in the fore end position of the displacement device 37, the change of the gripping means to a releasing position is made possible by means of the tension spring 41. As shown in Figs. 3 and 4 the cylindrical body has a pin 43 with a flange 44 and a coil spring 45 provided to hold the pin 43 in the position shown in Fig. 3 through which the gripping plate 12 will be held in the hooking position of the gripping means. In the lower part of the pin 43 is a metal socket 47 pressed against a projection 46, whereby contact of an electric circuit is achieved with a contact strip 49 against the metal socket 47 without this contact action being a stop position for the pin 43, thus being able to be further pressed down a little when contact has been achieved between the metal socket 47 and the contact strip 49. This switch is the main switch for the control device 19 and the motor 14. The switch is in a releasing position when the hypodermic syringe 4 is removed from the injection device but to be in an on-position according to Fig. 4 when the hypodermic syringe is in plce with the gripping plate 12 pressing down the pin 43.

The injection device 1 further has an upper supporting means located in the middle part 15 some distance down from the opening 3. The supporting means 50 are intended to form a support for the hypodermic syringe 4 in its upper part and thus to center this in the cavity 5 of the injection device and to automatically lift off the needle protection 13 before the syringe is loaded. The location of the supporting means are shown in Figs. 1 and 11 and its construction is shown in Figs. 5 and 6. The supporting means 50 are changeable between a retracted position shown in Figs. 1 and 5 and a protruded supporting position shown in Figs. 6, 11 and 12. In the retracted position the hypodermic syringe can be put in place into the injection device while in the protruded supporting position the hypodermic syringe is centered and gets support to its sides. However, the hypodermic syringe is not held but is able to make a displacement movement relative to the supporting means. This is done during the moment of insertion, further to be described later on. The supporting means are made of two supporting elements 51 which are movable from and against each other in grooves on the inside of the tubelike middle part 15. The two supporting elements 51 have in between them two coil springs 76, biassing the supporting elements away from each other in the retracted positions. On the upper side of the supporting elements are two metal strips 52 situated, intended to be a part of an electrical circuit to be further described later on. Included in the supporting means are also two long expansion means 53 in the form of electrically conductive bars which are electrically isolated from the tube-like middle part 15 and other parts of the device too. The two expansion means extend all down to the lower part of the injection device in the position shown in Fig. 1 and are fixed connected to a spring ring 54, which is displaceable within the bore of the house 14 relatively the house as well as the middle part 15 and the inner part 16. The spring ring 54 however is connected to the middle part 15 with two tension springs 55 of which one is shown in Fig. 1. Through this the ring 54 is brought to follow the outgoing movements of the middle part 15 during the main part of its displacement distance but is hooked through a hooking element 56 in the inner part 16 and remains in the position shown in Fig. 11, through which the two tension springs 55 are brought to be stretched during the last displacement movements of the middle part 15. As indicated in Fig. 11 with a partly broken view of one expansion means 53, these have an impact area which will be hooked against said means. When doing so the expansion means 53 halts in their displacement movements, through which the middle part 15 and thus also the supporting elements 51 move relative to the expansion means. As each of these in the upper ends are made with an oblique angled guiding area 57 this relative movement makes the two supporting means 51 to be pressed inwards against action of their coil springs 52 to the supporting position shown in Figs. 6 and 11, whereby the needle protection 13 thus is pressed upwards and through this will be lifted up and the hypodermic syringe will be stabilized and centered.

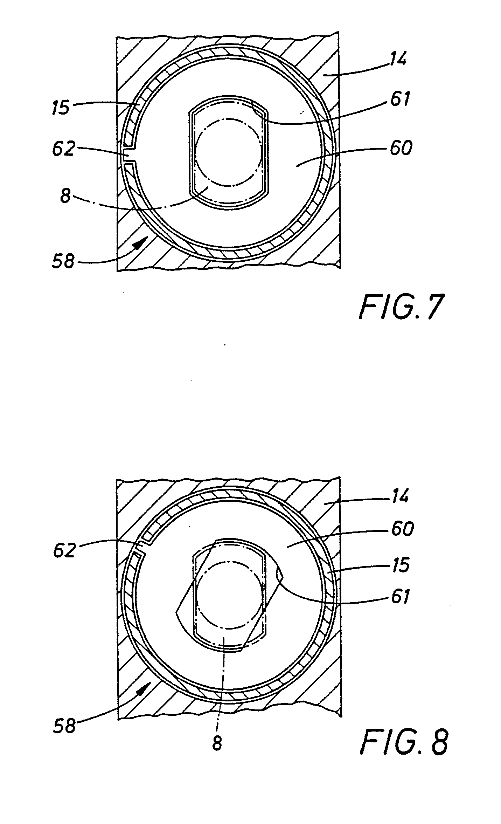

Upwards in the inner part 16 further on a holding means 58 for the barrel portion 6 of the hypodermic syringe is arranged. The holding means 58 is made of a narrowed part of the inner part 16 which forms stopping means 59 against which the hypodermic syringe 4 is put in place with its two laterally directed flanges in the flange part 8. Also the holding means 58 is provided with a holding means in the form of a twist ring 60, which can rotate in the inner portion 16. The construction of the holding means 58 is best shown in views of Figs. 7 and 8 where Fig. 7 shows the holding means in a releasing position and Fig. 8 shows the holding means in a holding position. The twist ring 60 has a central hole 61 which mainly corresponds to the form of the portion of the barrel portion 6 of the hypodermic syringe with the two flanges in the flange portion 8 and somewhat exceeding the dimensions for these. Through this the flanges are permitted to be placed on the stopping means 59 and situated on a level located under the twist ring 60. The twist ring has in its periphery a guide screw 62, which penetrates in a long obliquely formed perforation in the tube-shaped middle portion 15.

The height of the perforation corresponds to the total displacement distance of the middle portion. In the releasing position shown in Fig. 7 the hole 61 of the twist ring 60 forms in a way that it coincides with the stopping means 59 for the flange portion 8. By displacement of the tube formed middle portion 15 relative the house 14 of the injection device a twisting motion occurs of the twist ring 60, since the inner portion 16 and thus also the twist ring 60 will not join in the outpulling movements and the guide screw 62 will be guided in the perforation in the middle portion. As soon as this motion begins an edge portion of the hole 61 will be moved over the flange portion 8 of the barrel portion of the hypodermic syringe, thus making the barrel portion of the hypodermic syringe to be held in the inner part 15.

Returning to reference to Fig. 1 that shows that the inner portion will be held in a position in Fig. 1 with a pawl 63, which has a rotatable bearing in the house 14 and can be activated from the outside against action of a spring with a trigger 64. The pawl 63 cooperates with an inner edge 65 down on the outside of the inner portion 16 as long as the trigger is not pushed. The upper pawl 32 has a similar trigger 66, which can be pushed in against action of a spring for releasing of the pawl 32, which happens when the middle portion shall be brought back to its retracted position. This will be done manually after the injection device has performed its operations. Also the injection device has on the upper portion of the middle portion 15 an adjustable ring 67, for insertion depth which is threaded on the middle portion and can be adjusted in different heights through rotation. Inside said ring 67 is a top cover 68 which constitutes the opening 3 of the injection device and has spring action acting on it so that it strives to maintain a top position shown in Fig. 1, but can be pushed down as long as the hypodermic syringe is located in its inner position. When pushing down the top cover 68 an electric circuit is closed in order to activate the motor 17 for injection, this achieved by contact strips or a microswitch located in the top cover.

Fig. 9 shows all schematically the construction of the electronical controlling mechanism 58 for controlling the electrical motor 17. The adjustable switches 21 and 22 are for example thumb wheel switches which are connected to inputs of an electronic sequence block 69 which can be constructed through conventional digital technique and being made to activate the electrical motor 17 for rotation with its outgoing shaft 23 in one or the other direction during a predetermined time in order to perform all the working movements. The motor 17 has a feed back control through e.g. an optical pulse giver 70, which brings back information of the numbers of rotation of the motor and consequently performed work. The optical pulse giver 70 is so constructed with a frequency divider that the feed back signal is divided down to a frequency that corresponds to a certain amount of pulses for every unit of insulin, e.g. one pulse per unit. The insulin dosage is viz. in number of units, thus four units correspond to one tenth of a millimetre, this usually called a line, since the hypodermic syringes are graded in such lines. To the sequence block 69 and the feed back circuit also a buzzer 71 is connected, which marks the amount of units that are sucked in during the loading moment, e.g. every fourth unit. To the input side of the sequence block 69 are three switches 73, 74 and 75 connected via delaying and adapting circuits 72. The three switches 73, 74 and 75 are situated in the injection device in order to start certain actions through mechanical activation. One switch 73 has been mentioned earlier and is situated in the displacement means 34 and is put on by putting in the hypodermic syringe 4, more exactly by pressing down the pin 43 with the gripping plate 12. The second switch 74 is put on when the displacement means 34 is in its back position and can be made by a microswitch or similar device. The third switch 75 has also been mentioned above and has been designed to close the electrical circuit through the expansion means 50 or when the top cover 68 is pushed in and when the trigger 64 is pulled simultaneously which activates the motor 17 for injecting. The sequence block can e.g. be made of a fourbit counter and is counting positions (10—15) to be decoded with a 16 exit demultiplexer. Each of these exits represent a time phase in the working cycle. They will be gated together and will control the motor via logic circuits. One counter is also arranged for every adjustable switch and is counted down to zero from the set value.

With reference to Figs. 1, 10, 11 and 12 the different actions of the use of the displacement device 1 will be described. The initial position of the injection device is shown in Fig. 10 before any hypodermic syringe has been put into the device. At this stage the tube formed middle portion 15 is in a totally retracted position and the driving joggle 30 thus in its lower position.

The displacement means 34 is in its upper position at this moment thus gripping means 37 in its releasing position (see Fig. 3). Since the middle tube 15 is in its inner position the holding means 58 also is in a release position and the supporting means 50 in retracted position. All three switches 73, 74, 75 are in this moment in non-closed position. When a hypodermic syringe 4 is put into the injection device through the opening 3 to the moment when its flange portion 8 comes in contact against the stopping means 59 (see Fig. 1), the pin 43 is pressed down in the displacement means 34 (see also Fig. 4), making the switch 73 to close. At this moment the motor 17 is started in such a direction, that the driving joggle 30 is displaced upwards at the same moment as the displacement means moves inwards, i.e. downwards. At the same moment the gripping means 37 change at once to a holding position through the movements of the displacement means, bringing with the gripping plate 12 in direction backwards and the plunger 10 to be displaced downwards. Fig. 1 shows this position when the middle tube 15 has been lifted some distance by the driving joggle 30 and the displacement means 34 is moved some distance downwards. That the plunger follows and the displacement means 34 moves downwards in this first movement has however no function, since the loading action has not begun. This first movement has the only purpose to bring with the long expansion means 53 and the ring 54 with the tension spring 55. Some distance before the upper end position for the middle portion 15 the expansion means 53 are hooked and thus also the ring 15 through the hooking part 56 on the inner part 16 (see Fig. 11), this making the ring and the expansion means to stop in their upward going movement, while the middle portion 15 continues the last distance. At this movement the two supporting elements 51 of the supporting means 50 are changed from their retracted position to their protruded positions in order to center the hypodermic syringe through cooperation with the oblique edges 57 of the expansion means 53. During the pushing out movement of the middle portion 15 the displacement means 58 also are changed from its releasing position to its holding position according to Fig. 8. In its outer position the middle portion is hooked up through the pawl 32 and remains thus in this position. In this end position the switch 74 is activated, making the controlling means 19 to activate and switch the rotating direction of the motor 14.

The switch 74 can be a switch that e.g. is situated in the bottom of the inner portion 16 and to be activated of the displacement means 34 which at this moment is in its lower end position according to Fig. 11.

At this moment the displacement means 34 moves back to its fore position shown in Fig. 10, which is the starting point for the loading moment. This begins by the displacement means 34 moves in direction downwards to a position that corresponds to the preset insulin volume, set by the wheel 21. A mainly circular detachable bottle holder 69 is put above the top cover 68 and has a round opening 70, through which an insulin bottle 71 turned down can be put inside in order for the hypodermic needle 7 to penetrate the rubber membrane in the end of the bottle. At this moment the switch 75 is closed which e.g. can be the two metal strips 52 earlier mentioned and the metallic top 72 at the membrane of the insulin bottle 71. For practical reasons this however will be replaced by a microswitch or something similar because of the fact that not all bottles have an electrically conductive surface. At this moment the controlling means 19 is activated in order to start the motor 14 making the displacement means to move upwards to its upper end position, thus the insulin bottle 71 will be filled with an amount of air corresponding to the amount of insulin to be injected, which creates a favourable excess of pressure. When the displacement means 34 has reached its fore position and thus also the plunger its upper position, the rotation direction is switched after which the displacement means 34 is pulled down to a position that corresponds to the preset insulin volume plus additional volume corresponding e.g. to two units after which it turns back and stops in a position correspondong to the preset volume. During this movement thus insulin is sucked in and any possible encased air will be ejected, as shown in Fig. 11. The preset amount of insulin thus now is in the hypodermic syringe 4.

The loading action has now been finished, thus the insulin bottle 71 and the bottle holder 69 will be pulled away and the adequate depth of insertion is set by the adjustable ring for setting the insertion depth 67. After this the injection device is pushed against the body tissue 76 where the insulin is to be injected while the top cover 68 is pressed in. The top cover is pressed at this moment against action of its coil spring to the depth that the upper side of the adjustable ring for setting the insertion depth is resting against the skin. The device is now ready for insertion which occurs by pulling the trigger 64 at which under action of the tension springs 55 the inner portion 16 together with the hypodermic syringe 4 is inserted until the spring ring 54 comes in contact with its upper end position, that is determined of a contact portion 78 with which a part 79 of the spring ring cooperates (see Fig. 12). The hypodermic syringe with its needle thus penetrates to the necessary depth and through closure of the switch 75, which is achieved when the top cover 68 is pushed and the trigger 64 is pulled at the same time, the injection action occurs, that is that the motor 17 displaces the displacement means 34 and accordingly the plunger 10 of the hypodermic syringe 4 forwards in a way that the enclosed amount of insulin will be injected in the body tissue. At the same time the buzzer 71 can ring if so wanted. When the plunger 10 has reached its end position and all of the amount of insulin has been injected, the injection device is withdrawn and is placed on its previous place. The driving joggle 30 is in this moment in its lower end position, making it possible to release the middle part 15 from its hooked position by pushing the upper pawl 61. The middle portion can manually be pushed inside in the house 14, at which the barrel portion 6 of the hypodermic syringe will be released from its holding position, all done in a similar manner as has been previously described but in an opposite way. The gripping means 37 has also been changed to a releasing position making also the gripping plate free in a manner that the hypodermic syringe can be totally withdrawn from the injection device.

According to a favourable way of carrying out the invention, the top cover 68 also acts as a safety contact in order to prevent insertion of the injection device to occur when not intended. If projection is to occur the trigger must be pulled and the top cover must be pushed down in the same moment. A twisting disc is situated in the top cover over the upper portions of the expansion means 53 and prevents movements upwards of the expansion means as long as the top cover is not pushed inwards. This safety function can only be cancelled by a circular movement made by pushing in the top cover at which an oblique edge of the top cover acts on the twisting disc in order for the twisting disc to twist when the top cover is pushed in. At this moment a notch in the twisting disc will be situated at the middle of each upper end of the expansion means, in order to make it possible for the expansion means to freely be displaced upwards and projection can occur.

As mentioned above for the sake of simplicity, loading and injection of one kind of insulin has only been mentioned. Principally the injection device works in a similar manner at the use of two kinds of insulin. In this case the two dosages are set by the adjustable switches 21 and 22. Sucking in air in the hypodermic syringe occurs to a volume corresponding to the total amount of insulin, that is the sum of the amount of insulin for both kinds of insulin. The two kinds of insulin are provided each in an insulin bottle of which the first is placed in the upper part in the injection device and injection of air occurs corresponding to the amount of insulin for the first kind of insulin. After this the first bottle is withdrawn and the other bottle is attached. Then air corresponding to the amount of insulin for the other kind of insulin is injected in corresponding bottle. Then the set amount of insulin is sucked in to the hypodermic syringe from the second bottle, after which the bottle is withdrawn and the first bottle is put on. The amount of insulin for the bottle of insulin is after this sucked into the hypodermic syringe from the first bottle that then is withdrawn and the device is ready to inject in exactly the same manner as in the example described above.

The bottle holder shown in Figs. 13, 14 and 15 for the bottles 81 and 82 with two kinds of medicine e.g. two different kinds of insulin facilitates the loading procedure especially for blind people, since any error cannot happen, provided only that the bottles are in the right place in the bottle holder. This bottle holder has two cylindrical through going cavities 83, 84 made to enclose each a bottle. This cavity has an in steps narrowing portion 85, designed to form a support for the narrowing portion 86 of the bottle. Furthermore a tube formed portion 87 is projecting from the body of the bottle holder 80 by each cavity. This projecting portion has external threads and is slotted in order to create many elements 88, that in their upper ends have a narrowing diameter. Each tube formed portion 87 is surrounded of a ring 89, through which each bottle 81, 82 can be cramped by screwing up the ring after the bottles have been inserted. The in steps narrowing part 85 of the cavity has a ring formed supporting area 90 facing downwards. Through this supporting area the bottle holder is intended to rest against a supporting area facing upwards in the upper end of the injection device, as shown in Fig. 15. From this it is evident that also in this example an injection device with a slightly divergent construction is shown, compared with other figures. The narrowing part 85 of the holder 80 is so designed that it can project in the opening of the top cover and thus get support from its edge area. The holding ability of the bottle holder 80 is achieved also by four downwards projecting hooking means 91, 92, 93 and 94.

In a similar manner the upper portion of the injection device is formed with two notches, designed to fit to two hooking means at a time, that is either the hooking means 91, 92 or the hooking means 93, 94 depending on the bottle 81, 82 that is means to be used during the loading procedure. The two hooking means 91, 92 and 93, 94 respectively have different dimensions for each bottle. In a corresponding way the notches in the upper part of the injections device have different dimensions. Through this arrangement thus each bottle can be attached only in one way, which is important for the shape of the loading action. The third switch that has been described earlier is in this case replaced by two switches 95, 96 which are designed to close each a circuit through contact with any conducting part of the device. The two switches 95, 96 could e.g. be connected to each one of the two expansion means 93, at which the bottle holder 80 has another contact part that e.g. is situated in the ring formed supporting area 90 and is designed in a different way for each of the holding parts of the two bottles. The contact parts in the holder 80 are so situated that when attaching one bottle, that is the narrowing part 85, the switch 95 is activated but when attaching the other bottle the other switch 96 is activated. This can for example be arranged in a way that the supporting area 90 has over a part an electrically conducting cover, that reaches along the outside of the narrowing part 85 and achieves contact with e.g. an electrically conductive part of the top cover and through this activates the electrical controlling means 58 in a way that loading takes place to the right amount of insulin. At the narrowing part 85 for the other bottle the electrically conductive layer is placed at another part of the projecting surface 90 so that the other switch 96 will be activated and closing another circuit of activation of the electrical controlling mechanism for loading of the other kind of insulin.

The holder 80 for the two bottles 81, 82 are thus used in a way that first the bottle holder 80 is placed in a way that one bottle 81 will be situated right above the upper part of the injection device so that the hypodermic syringe can penetrate into the bottle and loading can occur with the first kind of insulin. After this the holder is withdrawn and moved away so that the other bottle of insulin 82 will be situated right above the hypodermic syringe and loading can occur with the other dosage of insulin. The two switches 95, 96 are arranged in a manner that at the moment of injection the top cover 68 will in a similar way as mentioned above close an electrical circuit in order to start the injection action through activation of the electrical controlling mechanism.

The invention is not limited to the examples described and shown above in the drawings, but can be varied within the scope of the following claims. For example it is possible that the electronic part, that is the controlling means 19 and the accumulator 20 are placed in a unit separate from the device and connected with the other part through a flexible cable. It is furthermore possible that the pulling out action of the middle tube is not performed by the electrical motor, but can also be done totally in a manual way, at which the motor thus only performs the displacement movements for the displacement device 34.

Under these circumstances only one threaded shaft is sufficient at which also the driving joggle 30 is left out. It is also possible that the electrical motor is of a linear type and situated in the bottom with the driving shaft coaxially with the middle part and the length axis of the inner part.

The depth of the insertion depth can be achieved in a different manner, for example through an adjustable screw that determines the lower end position for the top cover by stopping the top cover against the upper part of the screw. The holding means can be replaced by means that is movable radially from the wall of the inner part, they can be done in form of pins or something similar.

## Claims

1. Injection device for medical purposes, for injection by using a manual hypodermic syringe (4), the syringe including a barrel portion (6) which at one end has a syringe needle (7) and at the other end a flange (8), and a plunger portion (9) having a plunger (10) displaceable in the barrel portion, said plunger performing movements, enabling medicine to be drawn into or injected from the barrel portion, and having a gripping means connected with the plunger by means of a plunger rod (11), said injection device comprising an opening (3), through which the injection syringe (4) can be at least partly be introduced into a cavity (5), and an inner part (16) which is provided with a holding device (58) for the barrel portion (6), the holding device (58) being capable of holding the barrel portion (6) against movements in its longitudinal direction relative to said inner part; a displacement device for displacing the plunger portion relative to the barrel portion, the inner part being moveable between a rear position with the syringe needle (7) positioned inside an opening and an advanced position with the syringe needle pushed outside said opening in order to insert the needle into body tissue (73); and an outer part (14), in which an electric motor (17) is positioned, said electric motor being connected to said displacement device by means of a transmission mechanism (18), characterized in that said said opening through which the syringe can be introduced is said opening through which the syringe needle is advanced in use, the holding device is moveable into a releasing position, in which the barrel portion is released, the displacement device is capable of displacing the plunger portion in both directions relative to the barrel portion between a fore and a rear displacement position and in the fore displacement position is in a releasing position for the plunger portion and in the remaining displacement positions is capable of moving the plunger portion in either direction, and that the electric motor is controlled by means of an electronic control device, for moving the displacement device in a sequence determined by means of the control device for loading the injection syringe as well as insertion and injection.

2. Device according to claim 1, characterized in that a tubular intermediate part (15) is positioned and displaceable between the inner part (16) and the outer part (14) and provided to be extended relative to the inner and the outer part, that between the inner part and the intermediate part a spring mechanism (55) acts which is stretched in connection with said extension movements of the intermediate part and tends to move the inner part with the injection syringe (14) a distance from a rear position to a fore position and that the outer part includes a trigger mechanism (64) with a releasable pawl (63) having a locking position in which it engages the inner part in its rear position and a releasing position in which it allows the inner part to be displaced from its rear position to its fore position.

3. Device according to claim 2, characterized in that supporting means (50) are positioned at the top of the intermediate part (15) which the retracted position of the intermediate part are in a withdrawn position and in the extended position of the intermediate part are in an extended supporting position for the centering and support of the barrel portion (6) at its upper end.

4. Device according to claim 3, characterized in that the movement of the support means (50) between said withdrawn and extended positions is accomplished by the action of extension means (53, 54) which follow during a part of the expansion movement of the intermediate part.

5. Device according to claim 4, characterized in that said spring mechanism (55) is connected to one of the expansion means (54).

6. Device according to claim 1, characterized in that the displacement device (34) includes a stopping means (43) and hooking means (37), which are moved between the holding and the releasing positions of the plunger portion (10) by cooperation with a guide surface (42) in the inner part (16).

7. Device according to claim 1, characterized in that the device includes a holder (80) for at least two medicine bottles (81, 82), said holder being provided to be connected to the end of the injection device so that upon loading insertion of the needle takes place in one of the bottles, and that means (95, 96) are provided to activate the control device (19) by sensing the position of the holder so that loading takes place with a preset medicine volume of medicine.

## Patentansprüche

1. Einspritzvorrichtung für medizinische Zwecke unter Verwendung einer manuellen Injektionsspritze (4) mit einem zylindrischen Teil (6), der an einem Ende eine Injektionsnadel (7) und am anderen Ende einen Flansch (8) sowie einen Kolbenteil (9) mit einem in dem zylindrischen Teil verschiebbaren Kolben (10) aufweist, wodurch es möglich ist, ein Arzneimittel in den zylindrischen Teil einzuziehen und aus diesem Teil zu injizieren, und mit einer mit dem Kolben über eine Kolbenstange (11) verbundenen Greifvorrichtung, wobei die Einspritzvorrichtung eine Öffnung (3) enthält, durch die die Injektionsspritze (4) wenigstens teilweise in eine Höhlung (5) eingeführt werden kann, und mit einem inneren Teil (16), der mit einer Haltevorrichtung (58) für den zylindrischen Teil (6) versehen ist, wobei die Haltevorrichtung (58) in der Lage ist, den zylindrischen Teil (6) gegen Bewegungen in dessen Längsrichtung relativ zu dem inneren Teil zu halten, mit einer Verschiebungsvorrichtung für die Verschiebung des Kolbenteiles relativ zum zylindrischen Teil, wobei der innere Teil zwischen einer rückwärtigen Position, in der die Injektionsnadel (7) sich innerhalb einer Öffnung befindet, und einer vorgeschobenen Position, in der die Injektionsnadel aus der Öffnung ausgestoßen ist, um sie in ein Körpergewebe (76) einzusetzen, verschiebbar ist, und mit einem äußeren Teil (4), in dem ein Elektromotor (17) angeordnet ist, der mit der Verschiebungssvorrichtung über einen Übertragungsmechanismus (18) verbunden ist, dadurch gekennzeichnet, daß die Öffnung, durch die die Injektionsnadel eingeführt werden kann, diejenige Öffnung ist, durch die die Injektionsnadel im Gebrauch vorgeschoben wird, daß die Haltevorrichtung in eine Löseposition bewegbar ist, in der der zylindrische Teil freigegeben ist, daß die Verschiebungsvorrichtung in der Lage ist, den Kolbenteil in beide Richtungen relativ zu dem zylindrischen Teil zwischen einer vorderen und einer rückwärtigen Verschiebeposition zu verschieben, wobei die Verschiebungsvorrichtung in der vorderen Verschiebeposition sich in einer Löseposition für den Kolbenteil befindet und in den übrigen Verschiebepositionen in der Lage ist, den Kolbenteil in eine Richtung zu bewegen, und daß der Elektromotor durch eine elektronische Steuervorrichtung gesteuert wird, um die Verschiebungsvorrichtung in einer durch die Steuervorrichtung bestimmten Reihenfolge für das Füllen der Injektionsspritze und das Einsetzen und Injizieren zu bewegen.

2. Einspritzvorrichtung nach Anspruch 1, dadurch gekennzeichnet, daß ein rohrförmiger Zwischenteil (15) zwischen dem inneren Teil (16) und dem äußeren Teil (14) verschiebbar angeordnet und so vorgesehen ist, daß er relativ zu dem inneren Teil und dem äußeren Teil verlängert ist, daß zwischen dem inneren Teil und dem Zwischenteil ein Federmechanismus (15) wirkt, der in Verbindung mit den Längsbewegungen des Zwischenteils gespannt wird und den inneren Teil mit der Injektionsspritze (14) eine Strecke von der rückwärtigen Position zu einer vorderen Position zu bewegen bestrebt ist, und daß der äußere Teil einen Auslösemechanismus (64) mit einer Rastklinke (63) mit einer Verriegelungsposition enthält, in welcher sie den inneren Teil in seiner rückwärtigen Position erfaßt, und mit einer Freigabeposition, in welcher es dem inneren Teil ermöglicht wird, sich aus seiner rückwärtigen Position in seine vordere Position zu verschieben.

3. Einspritzvorrichtung nach Anspruch 2, dadurch gekennzeichnet, daß am Vorderteil des Zwischenteiles (15) eine Abstützvorrichtung (50) vorgesehen ist, die sich in der zurückgezogenen Position des Zwischenteiles in einer zurückgezogenen Position befindet und sich in der vorgeschobenen Position des Zwischenteils in einer vorgeschobenen Stütz-position zur Zentrierung und Abstützung des zylindrischen Teiles (6) an seinem oberen Ende befindet.

4. Einspritzvorrichtung nach Anspruch 3, dadurch gekennzeichnet, daß die Bewegung der Abstützvorrichtung (50) zwischen der zurückgezogenen und der vorgeschobenen Position durch die Wirkung von Vorschubvorrichtungen (53, 54) ausgeführt wird, die während eines Teiles der Vorschubbewegung des Zwischenteiles folgen.

5. Einspritzvorrichtung nach Anspruch 4, dadurch gekennzeichnet, daß der Federmechanismus (55) mit einer der Vorschubvorrichtungen (54) verbunden ist.

6. Einspritzvorrichtung nach Anspruch 1, dadurch gekennzeichnet, daß die Verschiebungsvorrichtung (34) eine Anhaltevorrichtung (43) und eine Greifvorrichtung (37) enthält, die durch Zusammenwirken mit einer Führungsfläche (42) im inneren Teil (16) zwischen der Halteposition und der Löseposition bewegt werden.

7. Einspritzvorrichtung nach Anspruch 1, dadurch gekennzeichnet, daß ein Halter (80) für wenigstens zwei Medikamentenflaschen (81, 82) vorgesehen ist, der mit dem Ende der Injektionsvorrichtung so verbindbar ist, daß beim Füllen ein Einsetzen der Nadel in eine der Flaschen erfolgt, und daß eine Vorrichtung (95, 96) vorgesehen ist, welche durch Abfühlung der Position der Halters die Steuervorrichtung (19) betätigt, so daß ein Füllen mit einem vorbestimmten Medikamentenvolumen erfolgt.

**Revendications**

1. Dispositif d'injection à usage médical, pour injection par utilisation d'une seringue hypodermique manuelle (4), la seringue incluant une portion formant corps cylindrique (6) qui présente à l'une de ses extrémités une aiguille de seringue (7) et à l'autre extrémité un flasque (8), ainsi qu'une portion formant plongeur (9) comprenant un plongeur (10) qui peut se déplacer dans la portion formant corps cylindrique, ledit plongeur effectuant des mouvements permettant d'aspirer un produit médicinal dans la portion formant corps cylindrique ou d'injecter ce produit médicinal depuis la portion formant corps cylindrique, et comprenant un moyen de saisie connecté avec le plongeur au moyen d'une tige de plongeur (11), ledit dispositif d'injection comportant une ouverture (3) par laquelle la seringue pour injection (4) peut être, au moins partiellement, introduite dans une cavité (5), et une partie intérieure (16) qui présente un dispositif de maintien (58) pour la portion formant corps cylindrique (6), le dispositif de maintien (58) étant capable de maintenir la portion formant corps cylindrique (6) à l'égard des mouvements dans sa direction longitudinale par rapport à ladite partie intérieure; un dispositif de déplacement pour déplacer la portion formant plongeur par rapport à la portion formant corps cylindrique, la partie intérieure pouvant se déplacer entre une position arrière, dans laquelle l'aiguille de la seringue (7) est placée à l'intérieur d'une ouverture, et une position avancée dans laquelle l'aiguille de la seringue est poussée à l'extérieur de ladite ouverture pour permettre d'insérer l'aiguille dans le tissu du corps (73); et une partie extérieure (14) dans laquelle est placé un moteur électrique (17), ledit moteur électrique étant connecté audit dispositif de déplacement au moyen d'une mécanisme de transmission (18), caracterise en ce que ladite ouverture, par laquelle on peut introduire la seringue, est ladite ouverture par laquelle on fait avancer l'aiguille de la seringue en service; en ce que le dispositif de maintien peut venir dans une position de déverrouillage dans laquelle la portion formant corps cylindrique est déverrouillée, la dispositif de déplacement est capable de déplacer la portion formant plongeur dans les deux directions par rapport à la portion formant corps cylindrique entre une position de déplacement avant et une position de déplacement arrière et, dans la position de déplacement avant, se trouve dans une position de déverouillage pour la portion formant plongeur et, dans les autres positions de déplacement, est capable de déplacer la portion formant plongeur dans l'une ou l'autre direction; et en ce que le moteur électrique est commandé au moyen d'un dispositif de commande électronique pour déplacer le dispositif de déplacement selon une séquence déterminée au moyen du dispositif de commande pour charger la seringue pour injection ainsi que pour l'insertion et l'injection.

2. Dispositif selon la revendication 1, caracterise en ce qu'une partie intermédiaire tubulaire (15) est placée et peut se déplacer entre la partie intérieure (16) et la partie extérieure (14) et en ce qu'elle est prévue pour s'étendre par rapoort à la partie intérieure et la partie extérieure; en ce qu'entre la partie intérieure et la partie intermédiaire agit un mécanisme à ressort (55) qui se bande en relation avec lesdits mouvements d'extension de la partie intermédiaire et tend à déplacer la partie intérieure, avec la seringue pour injection (14), sur une distance allant d'une position arrière à une position avant; et en ce que la partie extérieure comporte un mécanisme de déclenchement (64) avec un cliquet déverrouillable (63) qui présente une position de verrouillage dans laquelle il vient en contact de la partie intérieure dans sa position arrière et une position de déverrouillage dans laquelle il permet à la partie intérieure de se déplacer pour passer de sa position arrière à sa position avant.

3. Dispositif selon la revendication 2, caracterise en ce qu'en tête de la partie intermédiaire (15) sont placés des moyens supports (50) qui, dans la position rétractée de la partie intermédiaire, sont dans une position en retrait et, dans la position en extension de la partie intermédiaire, sont dans une position support en extension pour assurer le centrage et le support de la portion formant corps cylindrique (6) à son extrémité supérieure.

4. Dispositif selon la revendication 1 ou la revendication 3, caracterise en ce que le mouvement des moyens supports (50) entre ladite position en retrait et ladite position en extension s'accomplit sous l'action des moyens d'extension (53, 54) qui suivent durant une partie du mouvement d'expansion de la partie intermédiaire.

5. Dispositif selon la revendication 4, caracterise en ce que ledit mécanisme à ressort (55) est connecté à l'un des moyens d'expansion (54).

6. Dispositif selon la revendication 1, caracterise en ce que le dispositif de déplacement (34) comporte un moyen d'arrêt (43) et un moyen d'accrochage (37) qui se déplacent entre la position de maintien et la position de déverrouillage de la portion formant plongeur (10), par coopération avec une surface de guidage (42) de la partie intérieure (16).

7. Dispositif selon la revendication 1, caracterise en ce que le dispositif comporte un support (80) pour au moins deux flacons de produit médicinal (80, 82), ledit support étant prévu pour être connecté à l'extrémité du dispositif d'injection de façon que, lors du chargement, l'insertion de l'aiguille se fasse dans l'un des flacons; et en ce que des moyens (95, 96) sont prévus pour mettre en activité le dispositif de commande (19) en détectant la position du support de façon que le chargement se fasse avec un volume prédéter-miné de produit médicinal.

FIG.1

23
24
38 43
37
15
1
25 26 30 29

*FIG. 2*

42
37
43
44
45
48
36
38
49
47
46
41 34

*FIG. 3*

FIG. 4

FIG.5

FIG.6

FIG.7

FIG.8

FIG.9

FIG.10

*FIG.11*

FIG.12

FIG.13

FIG.15

FIG.14